# EUROPÄISCHE PATENTSCHRIFT

(11) **EP 0 808 342 B1**
(45) Veröffentlichungstag und Bekanntmachung des Hinweises auf die Patenterteilung: **30.12.1998**
(21) Anmeldenummer: 96901323.4
(22) Anmeldetag: 23.01.1996
(51) Int. Cl.: C08L 53/02, A61M 5/00

(54) **MEDIZINTECHNISCHES FORMTEIL, INSBESONDERE BAUTEIL FÜR EIN ÜBERTRAGUNGSSYSTEM FÜR INFUSIONS- ODER TRANSFUSIONSZWECKE**
MEDICAL MOULDED PART, IN PARTICULAR COMPONENT OF A TRANSMISSION SYSTEM FOR PERFUSION OR TRANSFUSION PURPOSES
PIECE MOULEE A USAGE MEDICAL, NOTAMMENT ELEMENT D'UN SYSTEME DE TRANSMISSION UTILE A DES FINS DE PERFUSION OU DE TRANSFUSION

(30) Priorität: 07.02.1995 DE 19503944
(43) Veröffentlichungstag der Anmeldung: 26.11.1997
(73) Patentinhaber: BASF AKTIENGESELLSCHAFT, 67056 Ludwigshafen (DE)
(72) Erfinder: NIESSNER, Norbert, D-67159 Friedelsheim (DE); KNOLL, Konrad, D-67069 Ludwigshafen (DE); NAEGELE, Paul, D-67166 Otterstadt (DE); WALTER, Hans-Michael, D-67251 Freinsheim (DE); KATHMANN, Jens-Otto, D-67063 Ludwigshafen (DE)
(86) Internationale Anmeldenummer: EP9600262
(87) Internationale Veröffentlichungsnummer: WO9624634

(56) Entgegenhaltungen:
- EP-A- 0 396 879
- EP-A- 0 512 530
- EP-A- 0 605 381
- WO-A-89/05837
- WO-A-95/35335
- CH-A- 637 148

## Beschreibung

Medizintechnische Formteile, insbesondere solche, die bei Übertragungssystemen für Infusions- und Transfusionszwecke und ähnliche medizinische Sonderanwendungen eingesetzt werden, sind Zubehörartikel, die schon lange in den unterschiedlichsten Ausführungen aus Kunststoffen im weitesten Sinne hergestellt worden sind; Kunststoffe in diesem Sinne sind auch die meisten kautschukelastischen Materialien.

Die aus thermoplastischen, gelegentlich auch aus duroplastischen Werkstoffen oder Elastomeren hergestellten Systeme oder Bestandteile sind im allgemeinen für die Einmalverwendung konzipiert, um Infektionen bei Mehrfachbenutzungen auszuschließen.

Einen Überblick über den Stand der Technik gibt die EP-A 7601, in der u.a. folgende Kunststoffe erwähnt werden: Polyvinylchlorid, Polyamid, Polystyrol, Polyethylen, Polypropylen, Polybuten, Polymethylpenten sowie Polyacrylsäureester und ferner Copolymere bzw. Blockcopolymere auf der Grundlage von Styrol-Butadien-Styrol, Styrol-Isopren-Styrol, Styrol-Acrylnitril, Acrylnitril-Butadien-Styrol, Styrol-Butadien, Methylmethacrylat-Butadien-Styrol, Methylacrylat-Styrol-Acrylnitril, Äthylen-Vinylacetat. Bei der vorgeschlagenen Verwendung von Styrol-Butadien-Blockcopolymerisaten wird allerdings nicht angegeben, um welche Polymerisate es sich dabei handeln soll, es ist aber offensichtlich, daß sich durchaus nicht alle oder auch nur die Mehrzahl der üblichen Blockcopolymerisate für jeden der angestrebten Zwecke eignet.

In der DE-A-22 16 987 sind neben Polyolefinen und PVC noch Polycarbonat und Celluloseester als Werkstoffe für die Medizintechnik erwähnt.

Wegen der Vielzahl der verwendeten Kunststoffe ist eine Entsorgung durch Verbrennung problematisch; insbesondere die Anwesenheit von Polyvinylchlorid bei der Verbrennung gilt als gefährlich, worauf die EP-A-396 879 hinweist.

Während für starre Bauteile Materialien zur Verfügung stehen, die sich problemlos entsorgen lassen und ggf. auch dem wiederverarbeitet (recycliert) werden können, besteht für Teile, die aus kautschukelastischem oder wenigstens schmiegsamem Material gefertigt sein müssen, nach wie vor ein Bedarf für ein Material, das die Verwendung des bewährten PVC ablösen kann.

Bei den bekannten Styrol-Butadien-Blockcopolymeren unterscheidet man zwei grundsätzlich verschiedene Werkstoffklassen:
a) Blockcopolymere mit Styrol als mengen- und volumenmäßigem Hauptanteil und einer untergeordneten Menge Butadien und
b) Blockcopolymere mit Butadien als mengen- und volumenmäßigem Hauptanteil und dafür geringerer Menge Styrol.

Beide Polymerklassen sind in Form von Handelsprodukten erhältlich. Gegenwärtig sind dies beispielsweise aus der Polymerklasse a) die Handelsprodukte Styrolux® (BASF) und K-Resin® (Phillips Petroleum). Beispiele für b) sind: Cariflex®, Kraton® (Shell) und Finaprene® (Petrofina) .

Polymerklasse a) sind transparente, schlagzähe Werkstoffe mit guter Verarbeitungsstabilität, da die thermisch vernetzbare Butadien-Monomereinheit nur im geringeren Anteil vorhanden ist. Diese Polymerklasse zeigt aber kein kautschukelastisches Verhalten, wie es für beispielsweise flexible Infusionsschläuche notwendig ist.

Polymerklasse b) sind ebenfalls in der Regel transparente Produkte, die für die Herstellung flexibler Formteile mit kautschukelastischen Eigenschaften verwendet werden. Diese Polymerklasse hat jedoch aufgrund ihres hohen Butadiengehalts den großen Nachteil der Thermoinstabilität, d.h. Formteile sind daraus nur unter besonderer Vorsicht mit speziellen Verfahren bei besonders niedrigen Temperaturen und unter besonders niedriger Scherbeanspruchung herzustellen. Bei den für beispielsweise die Schlauchextrusion üblichen Extrudertemperaturen von 200°C und darüber vernetzen und zersetzen sich diese Polymeren bereits.

Es besteht daher die Aufgabe, zwei an sich gegensätzliche Forderungen zu vereinen: Die Formmassen sollen einerseits thermostabil sein, d.h. bei einem Styrol-Butadien-Blockcopolymerisat müssen die Butadien-Monomereinheiten in deutlich geringerer Menge vorhanden sein als die Styrol-Monomereinheiten. Andrerseits soll die Formmasse kautschukelastisch sein, was nach dem Stand der Technik bedeutet, daß die Butadien-Phase im Überschuß vorhanden sein soll.

Der Erfindung lag weiterhin die Aufgabe zugrunde, Übertragungssysteme derart zu verbessern, daß sie vor allem umweltverträglich wiederverwertet werden können, wobei auf die Verwendung von insbesondere weichgemachtem PVC verzichtet werden soll. Demgemäß wurden die eingangs definierten Formteile gefunden. Bevorzugte Ausführungsformen sind den Unteransprüchen zu entnehmen.

Die Erfindung betrifft ein medizintechnisches Formteil, insbesondere ein Bauteil für ein Übertragungssystem für Infusions- oder Transfusionszwecke, aus einem Polymeren P1 oder einer Mischung aus P1, P2 und/oder P3, enthaltend
- P1:: 0.1-100 Gew.-% eines durch anionische Polymerisation erhaltenen kautschukelastischen Blockcopolymerisats P1 aus mindestens einem einpolymerisierte Einheiten eines vinylaromatischen Monomeren aufweisenden, eine Hartphase bildenden Block S und mindestens einem sowohl einpolymerisierte Einheiten eines vinylaromatischen Monomeren (S) wie eines Diens (B) aufweisenden elastomeren, eine Weichphase bildenden Block B/S, wobei die Polymerisation der Weichphase in Gegenwart eines polaren Cosolvens vorgenommen worden ist, derart, daß sie ein statistisches Copolymerisat des vinylaromatischen Monomeren mit dem Dien bildet und wobei die Glastemperatur T_{g} des Blocks S über 25°C und die des Blocks B/S unter 25°C liegt und das Phasenvolumen-Verhältnis von Block S zu Block B/S so gewählt ist, daß der Anteil der Hartphase am gesamten Blockcopolymerisat 1 bis 40 Volumen-% und der Gewichtsanteil des Diens weniger als 50 Gew.% beträgt
- P2:: 0 bis 99.9 Gew.-% eines thermoplastisch verarbeitbaren und/oder duroplastischen Polymeren P2,
- P3:: 0 bis 70 Gew.-% weiterer Zusatzstoffe und Verarbeitungshilfsmittel,
wobei die Summe der Gewichtsprozente der Komponenten P1 bis P3 100 % ergibt.

Überraschenderweise zeigen die erfindungsgemäßen Formteile neben einer guten Verarbeitbarkeit hervorragende Gebrauchseigenschaften. Hervorzuheben ist vor allem das exzellente Knickverhalten, was bei einem Styrolanteil von über 70 % nicht zu erwarten war.

Formteile der erfindungsgemäßen Art enthalten 0,1 bis 100, vorteilhaft 1 bis 99 und insbesondere 50 bis 99 Gew.-% des kautschukelastischen Blockcopolymerisats P1 und entsprechend 0 bis 99,9 vorzugsweise 1 bis 99 und insbesondere 1 bis 50 Gew.-% des thermoplastisch verarbeitbaren Polymeren P2 und sind i.a. noch dadurch besonders dadurch gekennzeichnet, daß T_{g} der Hartphase S des Blockcopolymerisats P1 über 50°C und T_{g} der Weichphase B/S unter 5°C liegt.

Das vinylaromatische Monomere des Blockcopolymerisats P1 ist vorzugsweise ausgewählt aus Styrol, a-Methylstyrol, Vinyltoluol und Diphenylethylen und das Dien aus Butadien und Isopren. Bevorzugt ist das erfindungsgemäß verwendbare Blockcopolymerisat P1 ein Styrol-Butadien-Blockcopolymerisat.

Die zu sog. lebenden Polymeren (living polymers) führende anionische Polymerisation, bei der das Wachstum eines Kettenmoleküls an einem Kettenende stattfindet, das mangels spontaner Kettenabbruch- oder übertragungsreaktion theoretisch beliebig lange lebt (polymerisationsfähig bleibt), und die Umsetzung des lebenden Polymeren mit ein- oder mehrfunktionellen Reaktionspartnern bietet bekanntlich eine vielseitig verwendbare Möglichkeit zum Aufbau von Blockcopolymeren, wobei die Auswahl an Monomeren allerdings beschränkt ist; in der Praxis haben nur Blockcopolymere von vinylaromatischen Verbindungen, also Styrol und seinen Abkömmlingen einerseits und Dienen, im wesentlichen Butadien oder Isopren andererseits Bedeutung erlangt. Blockcopolymere erhält man dadurch, daß jeweils bis annähernd zur Erschöpfung eines Monomerenvorrats polymerisiert und das oder die Monomeren dann gewechselt werden. Dieser Vorgang ist mehrfach wiederholbar. Das Eigenschaftsprofil dieser Blockcopolymeren wird wesentlich durch den Gehalt einpolymerisierter Dienmonomerer, d.h. Länge, Anordnung und Mengenverhältnis von Polydien- und Polystyrol-Blöcken geprägt. Darüberhinaus spielt die Art und Weise des Übergangs zwischen unterschiedlichen Blöcken eine wichtige Rolle: Man kennt scharfe und sog. verschmierte (tapered) Übergänge, je nachdem, ob der Monomerenwechsel abrupt oder allmählich stattfindet. Im letzteren Fall tritt eine mehr oder weniger statistische Sequenzlängenverteilung auf.

Blockcopolymere mit scharf getrennten Blöcken sind bei identischem Molekulargewicht und Dienanteil weniger zäh als solche mit verschmiertem Blockübergang. Will man zu zäheren Blockcopolymeren gelangen, wird man folglich Blockübergänge mit statistischer Sequenzlängenverteilung von Dien- und Vinylaromaten im Übergangsbereich bevorzugen (vgl. US-PS 4 122 134 und EP-A-0 316 671).

Bei morphologischen Untersuchungen von Blockcopolymeren zeigt sich nun, daß bei verschmiertem Blockübergang die Sequenz länge der reinen Dienphase gegenüber der Polystyrolphase und somit das Volumenverhältnis zugunsten der Dienphase verschoben ist. Durch die Art des Blockübergangs läßt sich also die Zähigkeit eines Polymerisats steigern, ohne daß der Diengehalt erhöht werden muß. Dies kann vorteilhaft sein, da mit wachsendem Diengehalt die Fließfähigkeit der Schmelze und die Thermostabilität der Polymeren abnimmt und die Gefahr der Vernetzung der Dienphase zunimmt, Bei Spritzguß- und Extrusionsverarbeitung macht sich die Vernetzung durch sog. Stippen und Trübungen im Polymerisat bemerkbar.

Die Erzielung verschmierter Blockübergänge durch gesteuerten Wechsel der Monomerenzugabe ist nun technisch aufwendig und führt zu einer längeren Reaktionsdauer bzw. geringerer Raum-Zeit-Ausbeute, was die Herstellungskosten erhöht. Im Grenzfall, der kontinuierlich gesteuerten Zugabe (vgl. US-PS 4 346 198 und 4 248 984) nimmt die Umsetzungsdauer wegen der ungünstigen Lage der Copolymerisationsparameter von Vinylaromaten und Dienen extrem zu und man gewinnt nur Polymere mit inhomogener Verteilung der Dien-und Vinylaromaten-Einheiten im Bereich des Blockübergangs, was sich wie eine Vermehrung der Zahl der Übergänge auswirkt. Deutlich wird dies durch eine niedrige Glastemperatur (T_{g} unterhalb von -50°C, vgl. US-PS 4 346 198, Beispiel 1) und schlechte Verarbeitungseigenschaften.

Besonders Materialien mit einem Dien-Gehalt von über 35 Gew.%, die aufgrund ihres Eigenschaftsprofils (Zähigkeit, Transparenz, Gasdurchlässigkeit) für medizintechnische Anwendungen wie Infusionsschläuche, Infusionstropfkammern und Dehnfolien geeignet wären, sind nur sehr schwierig durch Profilextrusion, Spritzguß oder Schlauchfolienextrusion zu verarbeiten; sie sind auch trotz Stabilisierung mit Antioxidantien und Radikalfängern thermisch sehr empfindlich und neigen zur Klebrigkeit, so daß man sich aufwendig mit Additiven behelfen muß. Das sog. Blocken (Verkleben von Fclien und Schläuchen auf der Rolle) und schlechte Entformbarkeit können die Verarbeitung durch Spritzguß gänzlich unmöglich machen.

Die erfindungsgemäß verwendbaren, sich kautschukelastisch verhaltenden Blockcopolymeren sollen großtechnisch einfach herstellbar sein, bei niedrigem Diengehalt ein Maximum an Zähigkeit besitzen und darüberhinaus wie Thermoplaste auf Extrudern und Spritzgußmaschinen einfach zu verarbeiten sein.

Erfindungsgemäß wird dies, allgemein ausgedrückt, dadurch möglich, daß man in einem Vinylaromat-Dien-Blockcopolymerisat aus Blöcken, die eine Hartphase (Blocktyp S) und solchen, die eine Weichphase bilden, an die Stelle eines reinen Polydienblocks als Weichphase einen Block B/S aus Dien- und Vinylaromaten-Einheiten treten läßt, der statistischen Aufbau besitzt. Der Aufbau kann dabei entlang der Kette im statistischen Mittel homogen oder inhomogen sein.

Man erhält ein solches erfindungsgemäßes kautschukelastisches Blockcopolymerisat dadurch, daß im Rahmen der oben angegebenen Parameter die Weichphase aus einen statistischen Copolymerisat eines Vinylaromaten mit einem Dien gebildet wird; statistische Copolymerisate von Vinylaromaten und Dienen erhält man im allgemeinen durch Polymerisation in Gegenwart eines polaren Cosolvens.

Bevorzugte erfindungsgemäße Blockcopolymerisate können durch eine der allgemeinen Formeln 1 bis 11 dargestellt werden:

(1) (S-B/S)ₙ;

(2) (S-B/S)ₙ-S;

(3) B/S-(S-B/S)ₙ;

(4) X-[(S-B/S)ₙ]ₘ₊₁;

(5) X-[(B/S-S)ₙ]ₘ₊₁;

(6) X-[(S-B/S)ₙ-S]ₘ₊₁;

(7) X-[(B/S-S)ₙ-B/S]ₘ₊₁;

(8) Y-[(S-B/S)ₙ]ₘ₊₁;

(9) Y-[(B/S-S)ₙ]ₘ₊₁;

(10) Y-[(S-B/S)ₙ-S]ₘ₊₁;

(11) Y-[(B/S-S)ₙ-B/S]ₘ₊₁;

wobei S für einen vinylaromatischen Block,
B/S für die Weichphase, den statistisch aus Dien- und vinylaromatischen Einheiten aufgebauten Block steht,
X den Rest eines n-funktionellen Initiators,
Y den Rest eines m-funktionellen Kopplungsmittels
und m, n natürliche Zahlen von 1 bis 10 bedeuten.

Insbesondere bevorzugt sind Blockcopolymerisate der allgemeinen Formeln S-B/S-S, X-[-B/S-S]₂ und Y-[-B/S-S]₂ (Bedeutung der Abkürzungen wie vorstehend) und besonders bevorzugt ein Blockcopolymerisat, dessen Weichphase unterteilt ist in Blöcke

(12) (B/S)₁-(B/S)₂;

(13) (B/S)₁-(B/S)₂-(B/S)₁;

(14) (B/S)₁-(B/S)₂-B/S)₃;

deren Vinylaromat/Dien-Verhältnis in den einzelnen Blöcken B/S unterschiedlich ist oder sich innerhalb eines Blocks in den Grenzen (B/S)₁≤(B/S)₂ kontinuierlich ändert, wobei die Glasübergangstemperatur T_{g} jedes Teilblocks unter 25°C liegt.

Ein Blockcopolymerisat, das mehrere Blöcke B/S und/oder S mit unterschiedlicher Molmasse je Molekül aufweist, ist ebenfalls bevorzugt.

Ebenso kann an die Stelle eines ausschließlich aus vinylaromatischen Einheiten aufgebauten Blocks S ein Block B treten, da es insgesamt lediglich darauf ankommt, daß ein kautschukelastisches Blockcopolymerisat gebildet wird. Solche Copolymerisate können z.B. die Struktur (15) bis (18) haben

(15) B-(B/S)

(16) (B/S)-B-(B/S)

(17) (B/S)₁-B-(B/S)₂

(18) B-(B/S)₁-(B/S)₂.

Erfindungsgemäße Blockcopolymerisate eignen sich außer für den speziellen erfindungsgemäßen Zweck hervorragend zur Herstellung von kautschukelastischen Formteilen mit den üblichen Methoden der Thermoplastverarbeitung, z.B. als Folie, Schaum, Thermoformling, Spritzgußformling oder Profilextrudat.

Zum Verständnis der nachstehenden Darstellung eines erfindungsgemäßen Übertragungssystems sei vorab geschickt, daß folgende Kriterien ausnahmslos erfüllt werden müssen:
- Hohe Transparenz des gesamten Überleitgerätes, z.B. zur besseren Kontrolle der Tropfkammerfüllung (Luftfallenwirkung), Einstellmöglichkeit der Tropffolge als Kriterium der Flüssigkeitsmenge pro Zeiteinheit.
- Sterilisierbarkeit (Be- und Entgasungsfreudigkeit gegen beispielsweise Ethylenoxid durch hohe Gasdurchlässigkeit)
- Flexibilität und Rückstellvermögen sowohl des Tropfkammerunterteils als auch aes Schlauches.
- Knickfestigkeit des Schlauches; bei einer typischen Schlauchgeometrie von 3mm Innen- und 4 mm Außendurchmesser darf bei Biegeradien bis herab zu etwa 2 cm noch kein Knick auftreten.
- Nach Abknickung mit einem Biegeradius von weniger als 2 cm müssen die Knickstellen sich wieder zurückbilden, so daß keine Knickstelle mehr sichtbar ist.
- Beständigkeit gegen energiereiche Strahlen bis zu 2,5 MRAD.
- Integrierte Anordnungsmöglichkeit von Schlauchansatz und Einstechdorn am Tropfkammer-, Unter- bzw Oberteil.
- Verschweiß- bzw. Verklebbarkeit und Umspritzung für Tropfkammer-Ober- und Unterteil bzw. aller Schlauchverbindungen mit Kegelverbindungsprinzip zur Erzielung einer flüssigkeits-und luftdichten Verbindungsstelle.
- Hohe Schlagzähigkeit der mechanisch beanspruchten Bauteile (z.B. bei plötzlicher Druckbeaufschlagung).

Zur Gewährleistung dieser Kriterien sollte ein derart oder in abgewandelter Form gestaltetes Übertragungssystem insbesondere aus einem Schlauch aus dem erfindungsgemäßen Blockcopolymerisat, gegebenenfalls als Mischung mit einem der nachstehend unter P2 und/oder P3 aufgeführten Mischungspartner bestehen. Die übrigen Teile können aus bekannten Werkstoffen, wie zum Beispiel schlagfestem Polystyrol, glasklarem Polystyrol, steifen bzw. schlagzähen Werkstoffen, z.B. ebenfalls aus der Klasse der Styrol-Butadien-Blockcopolymeren (z.B. Handelsprodukte wie Styrolux® (BASF), K-Resin® (Phillips Petroleum) oder Finaclear® (Petrofina)) oder aber aus einem Werkstoff bestehen wie Polypropylen, ABS, Polyethylen, welcher die genannten Kriterien erfüllt. In einer weiteren, bevorzugten Ausführungsform besteht das Tropfkammerunter-und/oder -oberteil aus dem vorgenannten Blockcopolymerisat oder einer Mischung mit einem weiteren Polymerwerkstoff.

Die Innendurchmesser der erfindungsgemäßen Infusions- und Transfusionsschläuche beträgt üblicherweise von 0,5 bis 5 mm, bevorzugt von 1 bis 3 mm. Die Wandstärken betragen üblicherweise von 0,1 bis 2 mm, bevorzugt von 0,2 bis 1 mm, besonders bevorzugt von 0,3 bis 0,6 mm.

Die erfindungsgemäßen Tropfkammern besitzen exzellentes Knickverhalten, einen hohen Rückstelieffekt und gleichzeitig mechanische Festigkeit. Die Tropfkammern können sowohl aus dem reinem Polymer als auch aus Mischungen mit weiteren geeigneten Polymeren hergestellt werden, bevorzugt Mischungen mit Styrol-Polymerisaten, besonders bevorzugt mit Polystyrol und Styrol-Butadien-Blockcopolymerwerkstoffen wie die vorgenannten Handelsprodukte styrolux®, K-Resin®, Finaclear®. Diese Werkstoffe, die selbst kein kautschukelastisches oder nur geringfügiges kautschukelastisches Verhalten zeigen, modifizieren das Blockcopolymerisat dahingehend, daß bei etwas reduzierter Kautschukelastizität die Festigkeit deutlich verbessert wird. Das führt zu einem "festeren Griff" des Tropfkammerteils, wie man es beispielsweise vom Polyvinylchlorid gewöhnt ist.

Die Erfindung umfaßt außer Formteilen vor allem auch transparente, flexible Schläuche mit exzellenten Knickverhalten, die nicht für Infusions- oder Transfusionszwecke verwendet werden. Beispiele hierfür sind sogenannte "heavy tubes" wie sie für Vakuumsaugvorrichtungen in der Medizintechnik verwendet werden, beispielsweise bei Behandlung eines Pneumothorax oder bei Absaugpumpen für den Mund/Rachenbereich.

Solche Schläuche werden heute in der Regel aus Polyvinylchlorid hergestellt, welches bislang als einziges Polymer die Anforderungen an das Knickverhalten, die Transparenz und die Verarbeitbarkeit bei hoher Druckstabilität erfüllt. Der Innendurchmesser solcher heavy tubes aus erfindungsgemäßem Material beträgt vorzugsweise von 5 bis 50 mm, bevorzugt von 10 bis 30 mm. Die Wandstärken betragen üblicherweise von 1 bis 15 mm, bevorzugt von 1,5 bis 10 mm, besonders bevorzugt von 2 bis 6 mm.

Erfindungsgemäße Teile sind auch Blutbeutel. Blutbeutel dienen dazu, das von einem Spender gelieferte Blut (in der Regel 500 ml) aufzufangen und unter Kühlung über einen Zeitraum von mehreren Monaten sicher aufzubewahren. Blutbeutel werden nach dem Stand der Technik praktisch ausschließlich aus Polyvinylchlorid hergestellt, da es bislang keinen anderen Werkstoff gibt, der Eigenschaften wie: Kälteflexibilität, gute Flüssigkeitssperreigenschaften, Sterilisierbarkeit mit Ethylenoxid oder gamma-Strahlen, mechanische Druckfestigkeit und gute Dichtigkeit der Schweißnähte bei der Blutbeutelherstellung miteinander vereint.

Die erfindungsgemäßen Blockcopolymerisate P1 sowie deren Mischungen mit den nachstehend beschriebenen Polymeren P2 und/ oder P3 zeigten überraschenderweise ein dem PVC vergleichbares Verhalten.

### Thermoplastisch verarbeitbare Polymere

Die Mischungspartner (Komponente P2) des erfindungsgemäßen kautschukelastischen Blockcopolymerisats P1 sind z.B. teilkristalline Polymerisate, vorzugsweise ausgewählt aus teilkristallinen Polyamiden, teilaromatischen Copolyamiden, Polyolefinen, Ionomeren, Polyestern, Polyetherketonen, Polyoxyalkylenen und Polyarylensulfiden.

### Polyamide

Teilkristalline, bevorzugt lineare Polyamide wie Polyamid-6; Polyamid-6,6; Polyamid-4,6; Polyamid-6,12 und teilkristalline Copolyamide auf Basis dieser Komponenten sind geeignet. Weiter können teilkristalline Polyamide eingesetzt werden, deren Säurekomponente ganz oder teilweise aus Adipinsäure und/oder Terephthalsäure und/oder Isophthalsäure und/oder Korksäure und/ oder Sebacinsäure und/oder Acelainsäure und/oder Dodecandicarbonsäure und/oder einer Cyclohexandicarbonsäure besteht, und deren Diaminkomponente ganz oder teilweise insbesondere aus m und/oder p-Xylylendiamin und/oder Hexamethylendiamin und/oder 2,2,4- und/ oder 2,4,4-Trimethylhexamethylendiamin und/oder isophorondiamin besteht, und deren Zusammensetzungen im Prinzip bekannt sind (vgl. Encyclopedia of Polymers, Vol. 11, S. 315 ff.).

Die Molekulargewichte Mₙ (Zahlenmittel) der als Komponente P2 geeigneten Polyamide liegen bevorzugt im Bereich zwischen 5,000 und 100,000, besonders bevorzugt zwischen 10,000 und 80,000.

Geeignet sind teilkristalline lineare Polyamide z.B. mit einer relativen Viskosität von 2,2 bis 4,5 (gemessen in 96 gew.-%iger Schwefelsäure bei einer Konzentration von 0,5 g/100 ml bei 25°C). Bevorzugt sind Polyamide, die sich ganz oder teilweise von Lactamen mit 7 bis 13 Ringgliedern ableiten, wie Polycaprolactam, Polycapryllactam oder Polylaurinlactam.

Geeignet sind ferner Polyamide, die durch Umsetzung von Dicarbonsäuren mit einem oder mehreren Diaminen erhalten werden. Geeignete Dicarbonsäuren sind beispielsweise Alkandicarbonsäuren mit 6 bis 12, insbesondere 6 bis 10 Kohlenstoffatomen, insbesondere Adipinsäure. Geeignete Diamine sind beispielsweise Alkan- oder Cycloalkandiamine mit 4 bis 12, insbesondere 4 bis 8 Kohlenstoffatomen; Hexamethylendiamin, m-Xylylendiamin, Bis(4-aminophenyl)methan, Bis(4-aminocyciohexyl)methan oder Bis(4-aminophenyl)propan-2,2 oder deren Mischungen sind besonders geeignete Partner zur Herstellung solcher Polyamide. Es kann vorteilhaft sein, die genannten Polyamide für sich herzustellen und deren Mischungen zu verwenden.

Besondere technische Bedeutung haben Polyamid-6 (Polycaprolactam), Polyamid-6,6 (Polyhexamethylenadipinamid) und Polyamide, die zu mindestens 80 Gew.-% aus wiederkehrenden Einheiten der Formel

-[-NH-(CH₂)₄-NH-CO-(CH₂)₄-CO-]-

aufgebaut sind. Die zuletzt genannten Polyamide sind durch Kondensation von 1,4-Diaminobutan mit Adipinsäure erhältlich. Geeignete Herstellungsverfahren für Polyamide sind z.B. in EP-A-38 094, EP-A-38 582 und EP-A-39 524 beschrieben.

Ebenfalls geeignet sind Polyamide mit geringem Anteil, vorzugsweise bis etwa 10 Gew.-%, an anderen einkondensierbaren Bestandteilen, insbesondere anderen Amidbildnern wie beispielsweise alpha, omega-Aminosäuren oder N-Carbonsäure-anhydriden (Leuchsanhydriden) von Aminosäuren.

Nach einer bevorzugten Ausgestaltung der Erfindung enthalten die erfindungsgemäßen Formmassen als Komponente P2 ein teilaromatisches Copolyamid mit dem nachstehend beschriebenen Aufbau.

Bevorzugte teilaromatische Copolyamide P2 enthalten als Komponente P21: 40 bis 90 Gew.-% Einheiten, die sich von Terephthalsäure und Hexamethylendiamin ableiten. Ein geringer Anteil der Terephthalsäure, vorzugsweise nicht mehr als 10 Gew.-% der Gesamtmenge an eingesetzten aromatischen Dicarbonsäuren kann durch Isophthalsäure oder andere aromatische Dicarbonsäuren ersetzt werden, vorzugsweise solche, in denen die Carboxylgruppen in para-Stellung stehen.

Neben den Einheiten, die sich von Terephthalsäure und Hexamethylendiamin ableiten, enthalten die teilaromatischen Copolyamide Einheiten, die sich von ε-Caprolactam ableiten (P22) und/oder Einheiten, die sich von Adipinsäure und Hexamethylendiamin (P23) ableiten.

Der Anteil an Einheiten, die sich von E-Caprolactam ableiten, beträgt bis zu 50 Gew. -%, vorzugsweise 20 bis 50 Gew.-%, insbesondere 25 bis 40 Gew.-%, während der Anteil an Einheiten, die sich von Adipinsäure und Hexamethylendiamin ableiten, bis zu 60 Gew.-%, vorzugsweise 30 bis 60 Gew.-% und insbesondere 35 bis 55 Gew.-% beträgt.

Die Copolyamide können auch sowohl Einheiten von ε-Caprolactam als auch Einheiten von Adipinsäure und Hexamethylendiamin enthalten; in diesem Fall beträgt der Anteil an Einheiten, die frei von aromatischen Gruppen sind, bevorzugt mindestens 10 Gew.-%, vorzugsweise mindestens 20 Gew.-%, Das Verhältnis der Einheiten, die sich von ε-Caprolactam und von Adipinsäure und Hexamethylendiamin ableiten, unterliegt dabei keiner besonderen Beschränkung.

Der Schmelzpunkt besonders geeigneter teilaromatischer Copolyamide liegt z.B. im Bereich von 260 bis über 300°C, wobei dieser hohe Schmelzpunkt auch mit einer hohen Glasübergangstemperatur von in der Regel mehr als 75, insbesondere mehr als 85°C verbunden ist.

Binäre Copolyamide auf der Basis von Terephthalsäure, Hexamethylendiamin und epsilon-Caprolactam weisen bei einem Gehalt von etwa 70 Gew.-% an Einheiten, die sich von Terephthalsäure und Hexamethylendiamin ableiten, einen Schmelzpunkt im Bereich von 300°C und eine Glasübergangstemperatur von mehr als 110°C auf.

Binäre Copolyamide auf der Basis von Terephthalsäure, Adipinsäure und Hexamethylendiamin erreichen bereits bei einem Gehalt von etwa 55 Gew.-% Einheiten aus Terephthalsäure und Hexamethylendiamin einen Schmelzpunkt von 300°C und mehr, wobei die Glasübergangstemperatur nicht ganz so hoch liegt wie bei binären Copolyamiden, die epsilon-Caprolactam anstelle von Adipinsäure bzw. Adipinsäure/Hexamethylendiamin enthalten.

Geeignete teilaromatische Copolyamide können nach den in den EP-A-129 195 und EP-A-129 196 beschriebenen Verfahren hergestellt werden.

Erfindungsgemäß können als Komponente P2 ferner amorphe Polyamide verwendet werden. Basierend auf den bereits genannten Monomeren werden noch zusätzliche, häufig mit einer oder mehreren, die Kristallisation behindernden Seitengruppen versehene Monomere einkondensiert. Als Resultat erhält man ein in der Regel transparentes Polyamid.

### Polyolefine

Beispiele für Polymerisate, die sich als Komponente P2 der erfindungsgemäßen Formmassen eignen, sind weiterhin teilkristalline Polyolefine, vorzugsweise Homo- und Copolymerisate von Olefinen wie Ethylen; Propylen; Buten-1; Penten-1; Hexen-1; Hepten-1; 3-Methylbuten-1; 4-Methylbuten-1; 4-Methylpenten-1 und Octen-1. Geeignete polyolefine sind demnach z.B. Polyethylen; Polypropylen; Polybuten-1 oder Poly-4-methylpenten-1. Allgemein unterscheidet man bei Polyethylen (PE) : High-Density-PE (HDPE), Low-Density-PE (LDPE) und Linear-Low-Density-PE (LLDPE).

Bevorzugt als Komponente P2 geeignete Polyolefine sind Polyethylen, Polypropylen und Poly-4-methylpenten-1, besonders bevorzugt Polyethylen und Polypropylen. Die Polyolefine können neben den Olefinen auch noch untergeordnete Mengen anderer Monomerer enthalten. Besonders geeignet sind z.B. Ethylen/Octen-Copolymere oder Ethyien/Hexen-Copolymere mit einem hohen Anteil von Octen oder Hexen (z.B. die Handelsprodukte Affinity® oder Engage® (DOW Chemical Co.))

### Ionomere

Bei einer anderen Ausführungsform der Erfindung handelt es sich bei der Komponente P2 um Ionomere. Dies sind im allgemeinen Polyolefine, wie sie vorstehend beschrieben wurden, insbesondere Polyethylen, die Monomere mit Carboxylgruppen einpolymerisiert enthalten, z.B. Acrylsäure, Methacrylsäure und ggf. weitere copolymerisierbare Monomere. Die Säuregruppen werden im allgemeinen mit Hilfe von Metallionen wie beispielsweise Na-, Ca-, Mg- und Al-Ionen in ionische, evtl. ionisch vernetzte Polyolefine umgewandelt, die sich jedoch noch thermoplastisch verarbeiten lassen (vgl. z.B. US-PSen 3,264,272; 3,404,134; 3,355,319; 4,321,337). Es ist jedoch nicht unbedingt erforderlich, die Säuregruppen enthaltenden Polyolefine mittels Metallionen umzuwandeln. Auch freie Säuregruppen enthaltende Polyolefine, die dann im allgemeinen einen kautschukartigen Charakter besitzen und teilweise noch weitere copolymerisierbare Monomere enthalten, z.B. (Meth)acrylate, sind als erfindungsgemäße Komponente P2 geeignet.

### Polyester

Daneben können als Komponente P2 auch Polyester, vorzugsweise aromatisch-aliphatische Polyester eingesetzt werden. Beispiele sind Polyalkylenterephthalate, z.B. auf Basis von Ethylenglykol, Propandiol-1,3, Butandiol-1,4, Hexandiol-1,6 und 1,4-Bis-hydroxymethylcyclohexan, sowie Polyalkylennaphthalate wie Polyethylen-oder Polybutylennaphthalat. Vorzugsweise werden Polyethylenterephthalat, Polybunylenterephthalat und Copolymere aus Cyclohexan-1,4-dimethanol, Ethylenglykol und Terephthalsäure eingesetzt. Insbesondere wird Polybutylenterephthalat verwendet.

Das Molekulargewicht (Gewichtsmittel, M_{w}) geeigneter Polyalkylenterephthalate liegt im allgemeinen zwischen 10,000 und 500,000, bevorzugt zwischen 10,000 und 80,000. Die Herstellung, z.B. durch Umesterung ist z.B. in den US-PSen 2,647,885; 2,643,989; 2,534,028 beschrieben.

### Polyetherketone

Als Komponente P2 können weiterhin aromatische Polyetherketone eingesetzt werden, wie sie z.B. beschrieben sind in der GB-PS 1 078 234, der US-PS 4,010,147, der EP-A-135 938 und in der Publikation von C. K. Sham et. al., Polymer 29/6, 1016-1020 (1988). Diese Polyetherketone können erhalten werden, indem man Bisphenole mit Bis(halogenaryl)ketonen in polaren aprotischen Lösungsmitteln in Gegenwart von Alkalicarbonaten, z.B. Lithiumcarbonat, umsetzt. Ein typisches derartiges Umsetzungsprodukt ist z.B. das aus Hydrochinon und 4,4'-Difluorbenzophenon gebildete Produkt.

### Polyoxyalkylene

Weiter können als Komponente P2 der erfindungsgemäßen Formmassen Polyoxymethylene eingesetzt werden, d.h. Polyoxyalkylene, die mindestens 50 mol-% wiederkehrende Einheiten -CH₂0- in der Polymerhauptkette aufweisen. Die Herstellung dieser Verbindungen ist allgemein bekannt: Die Homopolymeren werden im allgemeinen durch Polymerisation von Formaldehyd oder Trioxan in Gegenwart von geeigneten Katalysatoren hergestellt. Bevorzugt werden Oxymethylencopolymere, die neben den wiederkehrenden Einheiten -CH₂O-noch bis zu 50, vorzugsweise 0,1 bis 20 und insbesondere 0,3 bis 10 mol-% an wiederkehrenden Einheiten der Struktur (I)

-(̵-O-C(R¹,R²)-C(R³,R⁴)-(R⁵)ₙ-)̵- (I)

enthalten, wobei R¹ bis R⁴ unabhängig voneinander ein Wasserstoffatom, eine C₁-C₄-Alkylqruppe mit 1-4 C-Atomen und R⁵ eine -CH₂-, -CH₂O- eine C₁-C₄-Alkyl- oder C₁-C₄-Haloalkyl-substituierte Methylengruppe oder eine entsprechende Oxymethylengruppe darstellen und n den Wert 0 oder einen ganzzahligen Wert im Bereich von 1-3 annehmen kann. Vorteilhafterweise können diese Gruppen durch Ringöffnung von cyclischen Etnern in die Copolymere eingeführt werden. Als modifizierende 3austeine für die vorstehend erläuterten Polyoxyalkylene sind beispielsweise cyclische Ether geeignet wie Ethylenoxid, 1,2-Propylenoxid, 1,2-Butylenoxid, 1,3-Butylenoxid, 1,3-Dioxan, 1,3-Dioxolan und 1,3-Dioxepan sowie lineare Oligo- oder Polyformale wie Polydioxolan oder Polydioxepan.

Als Komponente P2 geeignet sind auch Oxymethylenterpolymerisate, die beispielsweise durch Umsetzung von Trioxan, einem der vorstehend beschriebenen cyclischen Ether und einem dritten Monomeren, vorzugsweise einem α-w-Diepoxid der Formel (II) 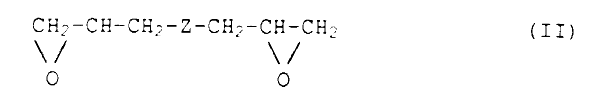 hergestellt werden, wobei Z eine Einfachbindung oder einen der zweiwertigen Reste -O-, (̵C₁-C₈-Alkylen)̵ oder (̵C₃-C₈-Cycloalkylen)̵ bedeutet.

Bevorzugte Monomere dieser Art sind Ethylendiglycid, Diglycidether und Diglycidylformal, Dioxan oder Trioxan im Molverhältnis 2:1 sowie Diether aus 2 Mol eines Glycidylrestes und 1 Mol eines aliphatischen Diols mit 2-8 C-Atomen wie beispielsweise die Diglycidether von Ethylenglykol, 1,2-Propandiol, 1,4-Butandiol, Cyclobutan-1,3-diol und Cyclohexan-1,4-diol.

Bevorzugte Oxymethylencopolymere haben einen Schmelzpunkt von mindestens 150°C und ein Molekulargewicht (Gewichtsmittel M_{w}) im Bereich von 5,000 bis 150,000, vorzugsweise von 7,000 bis 100,000. Endgruppenstabilisierte Oxymethylenpolymerisate, die an den Kettenenden C-C-Bindungen aufweisen, werden besonders bevorzugt.

### Polyarylensulfide

Als Komponente P2 sind weiter geeignet die Polyarylensulfide, insbesondere das Polyphenylensulfid. Dessen Herstellung ist beispielsweise beschrieben in US-PS 3,354,129, 3,786,035 und EP-A-171 021.

### Polyurethane

Als Komponente P2 der erfindungsgemäßen thermoplastischen Formmassen werden auch thermoplastische Polyurethane verwendet. Thermoplastische Polyurethane und Verfahren zu ihrer Herstellung sind bekannt und beispielsweise in der DE-A-36 28 562 beschrieben.

Geeignete thermoplastische Polyurethane können hergestellt werden z.B. durch Umsetzung von organischen, vorzugsweise aromatischen Diisocyanaten, Polyhydroxylverbindungen mit einem gewichtsmittleren Molekulargewicht M_{w} von 500 bis 8,000 und Kettenverlängerungsmitteln mit einem Molekulargewicht M_{w} von 60 bis 400. Als organische Diisocyanate kommen beispielsweise aliphatische, cycloaliphatische und vorzugsweise aromatische Diisocyanate in Betracht. Im einzelnen seien genannt: Aliphatische Diisocyanate, wie Hexamethylendiisocyanat, cycloaliphatische Diisocyanate, wie Isophorondiisocyanat; 1,4-Cyclohexandiisocyanat; 1-Methyl-2,4-cyclohexandiisocyanat und 2,6-cyclohexandiisocyanat sowie die entsprechenden Isomerengemische; 4,4'-; 2,4'- und 2,2'-Dicyclohexylmethandiisocyanat sowie die entsprechenden Isomerengemische und vorzugsweise aromatische Diisocyanate, wie 2,4-Toluylendiisocyanat, Gemische aus 2,4- und 2,6-Toluylendiisocyanat; 4,4'-; 2,4'- und 2,2'-Diphenylmethan-diisocyanat; Gemische aus 2,4'- und 4,4'-Diphenylmethan-diisocyanat; urethanmodifizierte, flüssige 4,4'- und/oder 2,4'-Diphenylmethandiisocyanate; 4,4'-Diisocyanatodiphenylethan-1,2 und 1,5-Naphthylendiisocyanat. Vorzugsweise verwendet werden Hexamethylendiisocyanat, Isophorondiisocyanat; 1,5-Naphthylendiisocyanat, sowie Diphenylmethandiisocyanat-Isomerengemische mit einem Gehalt von mindestens 96 Gew.-% an 4,4'-Diphenylmethandiisocyanat.

Als höhermolekulare Polyhydroxylverbindungen mit Molekulargewichten von 500 bis 8,000 eignen sich vorzugsweise Polyetherole und Polyesterole. In Betracht kommen jedoch auch hydroxylgruppenhaltige Polymere, beispielsweise Polyacetale, wie Polyoxymethylene und vor allem wasserunlösliche Formale, z.B. Polybutandiolformal und Polyhexandiolformal, und Polycarbonate, insbesondere solche aus Diphenylcarbonat und Hexandiol-1,6, hergestellt durch Umesterung, mit den oben genannten Molekulargewichten. Die Polyhydroxylverbindungen müssen zumindest überwiegend linear, d. h. im Sinne der Isocyanatreaktion difunktionell aufgebaut sein. Die genannten Polyhydroxylverbindungen können als Einzelkomponenten oder in Form von Mischungen zur Anwendung kommen.

Geeignete Polyetherole können dadurch hergestellt werden, daß man ein oder mehrere Alkylenoxide mit 2 bis 4 Kohlenstoffatomen im Alkylenrest mit einem Startermolekül umsetzt, das zwei aktive Wasserstoffatome gebunden enthält. Als Alkylenoxide seien z.B. genannt: Ethylenoxid, 1,2- Propylenoxid, 1,2- und 2,3-Butylenoxid. Vorzugsweise Anwendung finden Ethylenoxid und Mischungen aus Propylenoxid-1,2 und Ethylenoxid. Die Alkylenoxide können einzeln, alternierend nacheinander oder als Mischung verwendet werden. Als Startermolekül kommen beispielsweise in Betracht: Wasser, Aminoalkohole wie N-Alkyl-Diethanolamine (beispielsweise N-Methyldiethanolamin) und Diole, wie Ethylenglykol, 1,3-Propylenglykol, Butandiol-1,4 und Hexandiol-1,6. Es können auch Mischungen von Startermolekülen eingesetzt werden. Geeignete Polyetherole sind ferner die hydroxylgruppenhaltigen Polymerisationsprodukte des Tetrahydrofurans (Polyoxytetramethylenglykole).

Vorzugsweise verwendet werden Polyetherole aus Propylenoxid-1,2 und Ethylenoxid, in denen mehr als 50 %, vorzugsweise 60 bis 80 % der OH-Gruppen primäre Hydroxylgruppen sind und bei denen zumindest ein Teil des Ethylenoxids als endständiger Block angeordnet ist und insbesondere Polyoxytetramethylenglykole.

Solche Polyetherole können erhalten werden, indem man z.B. an ein Startermolekül zunächst Propylenoxid-1,2 und daran anschließend Ethylenoxid polymerisiert oder zunächst das gesamte Propylenoxid im Gemisch mit einem Teil des Ethylenoxids copolymerisiert und den Rest des Ethylenoxids anschließend anpolymerisiert oder schrittweise zunächst einen Teil des Ethylenoxids, dann das gesamte Propylenoxid und dann den Rest des Ethylenoxids an das Startermolekül anpolymerisiert.

Die für die Erfindung wichtigsten linearen Polyetherole besitzen ein Molekulargewicht von 500 bis 8,000, vorzugsweise 600 bis 6,000 und insbesondere 800 bis 3500. Sie können sowohl einzeln als auch in Form von Mischungen untereinander zur Anwendung kommen.

Geeignete Polyesterole können beispielsweise aus Dicarbonsäuren mit 2 bis 12 Kohlenstoffatomen, vorzugsweise 4 bis 8 Kohlenstoffatomen, und mehrwertigen Alkoholen hergestellt werden. Als Dicarbonsäuren kommen beispielsweise in Betracht: aliphatische Dicarbonsäuren, wie Bernsteinsäure, Glutarsäure, Adipinsäure, Korksäure, Azelainsäure und Sebacinsäure und aromatische Dicarbonsäuren, wie Phthalsäure, Isophthalsäure und Terephthalsäure. Die Dicarbonsäuren können einzeln oder als Gemische, z.B. in Form einer Bernstein-, Glutar- und Adipinsäuremischung, verwendet werden. Desgleichen sind Mischungen aus aromatischen und aliphatischen Dicarbonsäuren einsetzbar. Zur Herstellung der Polyesterole kann es gegebenenfalls vorteilhaft sein, anstelle der Dicarbonsäuren die entsprechenden Dicarbonsäurederivate, wie Dicarbonsäureester mit 1 bis 4 Kohlenstoffatomen im Alkoholrest, Dicarbonsäureanhydride oder Dicarbonsäurechloride zu verwenden. Beispiele für mehrwertige Alkohole sind Glykole mit 2 bis 10, vorzugsweise 2 bis 6 Kohlenstoffatomen, wie Ethylenglykol; Diethylenglykol; Butandiol-1,4; Pentandiol-1,5; Hexandiol-1,6; Decandiol-1,10; 2,2-Dimethylpropandiol-1,3; Propandiol-1,3 und Dipropylenglykol. Je nach den gewünschten Eigenschaften können die mehrwertigen Alkohole allein oder gegebenenfalls in Mischungen untereinander verwendet werden.

Geeignet sind ferner Ester der Kohlensäure mit den genannten Diolen, insbesondere solchen mit 4 bis 6 Kohlenstoffatomen, wie Butandiol-1,4 und/oder Hexandiol-1,6, Kondensationsprodukte von omega-Hydroxycarbonsäuren, beispielsweise omega-Hydroxycapronsäure und vorzugsweise Polymerisationsprodukte von Lactonen, beispielsweise gegebenenfalls substituierten omega-Caprolactonen.

Als Polyesterole vorzugsweise verwendet werden Dialkylenglykolpolyadipate mit 2 bis 6 Kohlenstoffatomen im Alkylenrest, wie z.B. Ethandiolpolyadipat; 1,4-Butandiolpoly-adipat; Ethandiolbutandiol-1,4-polyadipat; 1,6-Hexandiol-neopentylglykolpolyadipat; Polycaprolacton und insbesondere 1,6-Hexandiol-1,4-butandiolpolyadipat.

Geeignete Polyesterole besitzen z.B. ein Molekulargewicht M_{w} von 500 bis 6,000, vorzugsweise von 800 bis 3,500.

Als Kettenverlängerungsmittel mit einem Molekulargewicht M_{w} von 60 bis 400, vorzugsweise 60 bis 300, kommen vorzugsweise aliphatische Diole mit 2 bis 12 Kohlenstoffatomen, vorzugsweise mit 2, 4 oder 6 Kohlenstoffatomen, wie z.B. Ethandiol, Hexandiol-1,6, Diethylenglykol, Dipropylenglykol und insbesondere Butandiol-1,4 in Betracht. Geeignet sind jedoch auch Diester der Terephthalsäure mit Glykolen mit 2 bis 4 C-Atomen, wie z.B. Terephthalsäure-bis-ethylenglykol oder -bis-butandiol-1,4; Hydroxyalkylenether des Hydrochinons, wie z.B. 1,4-Di(β-hydroxyethyl)hydrochinon; (cyclo)aliphatische Diamine, wie z.B. 4,4'-Diaminodicyclohexylmethan; 3,3'-Dimethyl-4,4' -diaminodicyclohexylmethan; Isophorondiamin; Ethylendiamin; 1,2-; 1,3-Propylendiamin; N-Methylpropylendiamin-1,3; N,N'-Dimethylethylendiamin und aromatische Diamine, wie z.B. 2,4- und 2,6-Toluylendiamin; 3,5-Diethyl-2,4- und -2,6-Toluylendiamin und primäre ortho-di-; tri- und/oder tetra-alkylsubstituierte 4,4'-Diaminodiphenylmethane.

Zur Einstellung von Härte und Schmelzpunkt der thermoplastischen Polyurethane können die Polyhydroxyl-(Diol-)Verbindungen und Kettenverlängerungsmittel hinsichtlich ihres molaren Verhältnissen relativ weit variiert werden. Bewährt hat sich ein molares Verhältnisse von Polyhydroxylverbindung zu Kettenverlängerungsmittel von 1:1 bis 1:12, insbesondere von 1:1,8 bis 1:6,4, wobei die Härte und der Schmelzpunkt der thermoplastischen Polyurethane mit zunehmendem Gehalt an Polyhydroxylverbindung ansteigt.

Zur Herstellung der thermoplastischen Polyurethane werden die Aufbaukomponenten in einer solchen Menge umgesetzt, daß das Äquivalenzverhältnis von NCO-Gruppen der Diisocyanate zur Summe der Hydroxylgruppen oder Hydroxyl- und Aminogruppen der Polyhydroxylverbindungen und Kettenverlängerungsmittel 1:0,85 bis 1:1,20, vorzugsweise 1:0,95 bis 1:1,05 und insbesondere 1:0,98 bis 1:1,02 beträgt.

Geeignete Katalysatoren, welche insbesondere die Reaktion zwischen den NCO-Gruppen der Diisocyanate und den Hydroxylgruppen der Polyhydroxylverbindungen und Kettenverlängerungsmittel beschleunigen, sind die bekannten und üblichen tertiären Amine, wie z.B. Triethylamin, Dimethylcyclohexylamin, N-Methylmorpholin, N,N'-Dimethylpiperazin, 2-(Dimethylaminoethoxy)-ethanol, Diazabicyclo-(2,2,2)-octan (DABCO) und ähnliche sowie insbesondere organische Metallverbindungen wie Titansäureester, Eisenverbindungen wie z.B. Eisen-(III)-acetylacetonat, Zinnverbindungen, z.B. Zinndiacetat, Zinndioctoat, Zinndilaurat oder die Zinndialkylsalze aliphatischer Carbonsäuren wie Dibutylzinndiacetat, Dibutylzinndilaurat oder ähnliche. Die Katalysatoren werden üblicherweise in Mengen von 0,001 bis 0,1 Teilen pro 100 Teile Polyhydroxylverbindung eingesetzt.

### Sonstige Thermoplaste

Weiterhin als Komponente P2 geeignet sind selbstverstärkende kristalline Polyarylate (LCP's = liquid crystalline polymers), lineare Polyimide, Polybenzimidazole, Polyhydantoine, Polypyrrole, Polyphosphazene, Silicone.

### Duroplastische Polymere

Grundsätzlich können in die thermoplastischen Elastomeren P1 auch in duroplastische Werkstoffe P2 eingebaut werden, wie beispielsweise in Phenol-, Kresol-, Xylenol- und Resorcinharze, Harnstoff-und Melaminharze, Furanharze, vernetzte Polymethacrylate, ungesättigte Polyesterharze, Phenoacrylatharze, Epoxidharze, Isocyanatharze (Polyurethan-Vorprodukte) sowie sog. "Hybrid"-Präpolymere aus den vorstehend genannten Gruppen. Weiterhin als Mischungspartner P2 seien genannt: Allylesterharze; Polyurethane, z.B. halbharte RIM- (Reaction injection molding)-Teile, harte und halbharte Integralschaum-RIM-Systeme, harte und elastomere Polyurethan-Gießharze, harte bis weiche Schäume.

Die thermoplastischen Elastomeren P1 können weiterhin verwendet werden als Komponente in Prepregs (sheet molding compounds, SMC); bulk molding compounds (BMC) mit z.B. Polyester-, Phenacrylat-, Diallylphthalat- oder Siliconharz-Matrix; weiterhin in glasfaserverstärkten Matten (GFK), Halbzeugen und Fertigteilen.

### Nichtkristalline Polymerisate auf Acrylester- oder Styrolbasis

Als Komponente P2 der erfindungsgemäßen Mischung können mit besonderem Vorteil auch nicht-kristalline Copolymerisate wie Methacrylat-Acrylat-Styrol-Polymerisate, Acrylnitril-Butadien-Styrol-Polymerisate (ABS), Acrylnitril-Styrol-Acrylester-Polymerisate (ASA), Methacrylat-Butadien-Styrol-Polymerisate (MBS), schlagfestes Polystyrol (HIPS), aber auch Homopolymerisate wie Polystyrol, Polymethylmethacrylat (PMMA), Polyacrylnitril, Polymethacrylimid verwendet werden. Solche Polymerisate bestehen z.B. im Falle von ABS, ASA, MBS, aber auch im Falle von schlagfestem Polystyrol aus zwei Phasen, einer kontinuierlichen (oft als Hartphase oder Matrix bezeichnet) und einer dispersen (meistens als Kautschuk- oder Weichphase bezeichnet). Bei den einphasigen Polymeren fehlt in der Regel die disperse weiche Kautschukphase. Erfindungsgemäße Komponenten P2 sind somit einphasige Polymere, bestehend nur aus Hartphase (wie z.B. Polystyrol oder PMMA), zweiphasige Polymere (z.B. ABS, ASA, MBS, schlagfestes Polystyrol), aber auch nur die Weichphasen (z.B. der in ABS, ASA, MBS, schlagfestem Polystyrol enthaltene Kautschuk) in ungepfropfter, gepfropfter, reiner oder angereicherter Form.

Erfindungsgemäße schlagfeste Polystyrole und Standard-Polystyrole, deren Herstellung, Struktur und Eigenschaften sind in der Übersichtsliteratur (A.Echte, F.Haaf, J.Hambrecht in: Angew. Chem. (Int.Ed.Engl.) 20, 344-361, (1981); sowie Kunststoffhandbuch, Band Polystyrol, Carl Hanser Verlag (1968) eingehend beschrieben. Darüber hinaus können die verwendeten schlagzähen Polystyrole gegenüber üblichem schlagzähem Polystyrol strukturell verändert sein z.B. durch die Verwendung spezieller Polybutadienkautschuke. Solche speziellen Polybutadienkautschuke haben z.B. einen gegenüber herkömmlichen Kautschuken veränderten 1,4-cis-bzw. 1,4-trans-Anteil oder ein anderes Verhältnis von 1,2- und 1,4-Verknüpfungen. Ferner können anstelle von Polybutadienkautschuk auch andere Dienkautschuke sowie Elastomere von der Art des Ethylen-Propylen-Dien-Copolymer (EPDM-Kautschuk) sowie hydrierte Dienkautschuke eingesetzt werden. Geeignetes Standard-Polystyrol kann sowohl nach dem Verfahren der anionischen wie der üblicheren radikalischen Polymerisation hergestellt werden. Die durch das Polymerisationsverfahren beeinflußbare Uneinheitlichkeit des Polymerisats ist dabei von untergeordneter Bedeutung. Bevorzugt werden Standard-Polystyrol und schlagzähes Polystyrol, deren toluollöslicher Anteil ein Molekulargewicht M_{w} von 50,000 bis 500,000 g/mol aufweist.

Geeignete Monomere zur Bildung der Hartphase können beispielsweise aus den im folgenden aufgeführten Monomeren ausgewählt sein: Styrol und seine substituierten Derivate, wie z.B. alpha-Methylstyrol, 4-Methylstyrol, 3,4-Dimethylstyrol, 4-tert.-Butylstyrol, 2- und 4-Divinylbenzol, 4-Methyl-alpha-methylstyrol. und Technisch geeignet wären auch halogenierte Styrole wie alpha-Chlorstyrol, 4-Chlorstyrol, 2,4-Dichlorstyrol, 4-Chlor-alpha-Methylstyrol, die jedoch aus praktischen Gründen weniger infrage kommen. Bevorzugt sind Styrol und alpha-Methylstyrol.

Unter Acryl- und Methacrylverbindungen sind Monomere zu verstehen wie z.B. Acrylnitril, Methacrylnitril, Acryl- und Methacrylsäure, Methylacrylat, Ethylacrylat, n- und Isopropylacrylat, n- und Isobutylacrylat, t-Butylacrylat, 2-Ethylhexylacrylat, Methylmethacrylat, Ethylmethacrylat, n- und Isopropylmethacrylat, n- und Isobutylmethacrylat, t-Butylmethacrylat, Cyclohexylmethacrylat, Isobornylmethacrylat, Maleinsäureanhydrid und dessen Derivate, wie Maleinsäureester, Maleinsäurediester und Maleinimid, z.B. Alkyl- und Acrylmaleinimide, wie beispielsweise Methyl- oder Phenylmaleinimid, bevorzugt Acrylnitril, Methylmethacrylat, Maleinsäureanhydrid und Phenylmaleinimid.

Beispiele für geeignete Homo- bzw. Copolymerisate sind Polystyrol, Polymethylmethacrylat, Styrol-Acrylnitril-Copolymere, alpha-Methylstyrol-Acrylnitril-Copolymere, Styrol-Maleinsäureanhydrid-Copolymere, Styrol-Phenylmaleinimid-Copolymere, Styrol-Methylmethacrylat-Copolymere, Methylmethacrylat-Acrylnitril-Copolymere, Styrol-Acrylnitril-Maleinsäureanhydrid-Copolymere, Styrol-Acrylnitril-Phenylmaleinimid-Copolymere, alpha-Methylstyrol-Acrylnitril-Methylmethacrylat-Copolymere, alpha-Methylstyrol-Acrylnitril-t-Butylmethacrylat-Copolymere, Styrol-Acrylnitril-t-Butylmethacrylat-Copolymere.

### Polycarbonat

Erfindungsgemäße Komponenten P2 sind ferner beispielsweise Polycarbonat oder Mischungen aus Polycarbonat mit mehreren der nachstehend beschriebenen Pfropfcopolymeren und Thermoplasten. Bevorzugt liegt hierbei der Anteil an Polycarbonat zwischen 5 und 95 Gew.-% und der Anteil der Komponenten P24 + P25 zwischen 5 und 95 Gew.-%. Solche Mischungen sind im Handel erhältlich, beispielsweise unter der Handelsbezeichnung Bayblend® (Bayer) oder Terblend®S (BASF). Es handelt sich dabei um Mischungen eines Polycarbonats mit einem ABS- bzw. ASA-Polymerisat.

Geeignete Polycarbonate sind beispielsweise solche auf Basis von Diphenolen der allgemeinen Struktur H0-Ar-X-Ar-OH, worin Ar einen Arylenrest (Phenylen, Phenylalkyl, halogensubstituiertes Arylen) und X eine Einfachbindung, C₁-C₃-Alkylen, C₂-C₃-Alkyliden, C₃-C₆-Cycloalkyliden sowie -S- oder -SO₂- bedeuten.

Bevorzugte Diphenole der vorstehenden Struktur sind beispielsweise Hydrochinon; Resorcin; 4,4'-Dihydroxybiphenyl; 2,2-Bis(4-hydroxyphenyl)-propan; 2,4-Bis-(4-hydroxy-phenyl)-2-methylbutan; 1,1-Bis-(4-hydroxyphenyl)-cyclohexan. Besonders bevorzugt sind 2,2-Bis-(4-hydroxyphenyl)-propan und 1,1-Bis-(4-hydroxyphenyl)cyclo-hexan. Es handelt sich um bekannte Verbindungen und die sowohl einzeln als auch im Gemisch zur Herstellung der Polycarbonate verwendet werden können.

Geeignete Polycarbonate können in bekannter Weise verzweigt sein, und zwar vorzugsweise durch den Einbau von 0,05 bis 2,0 Mol-%, bezogen auf die Summe der eingesetzten Diphenole, an mindestens trifunktionellen Verbindungen, beispielsweise solchen mit drei oder mehr phenolischen OH-Gruppen.

Als besonders geeignet haben sich Polycarbonate erwiesen, die eine relative Viskosität nᵣₑₗ von 1,10 bis 1,50, insbesondere von 1,25 bis 1,40 aufweisen. Dies entspricht einem Molekulargewicht M_{w}, von 10,000 bis 200,000, vorzugsweise von 20,000 bis 80,000. Als Polycarbonate P2 im weiteren Sinne können auch Polycarbonat-Polysiloxan-Blockcopolymere, Polycarbonat-Polyether-Blockcopolymere und Polycarbonat-Polyester-Blockcopolymere dienen.

### Weitere Thermoplaste vom Polycarbonat-Typ

Erfindungsgemäß sind weiter als Thermoplaste P2 aromatische Polyester und Polyestercarbonate einsetzbar. Sie bestehen aus mindestens einem aromatischen Bisphenol der allgemeinen Formel (I) aus mindestens einer aromatischen Dicarbonsäure und gegebenenfalls aus Kohlensäure bzw. aeren Derivate, wie Phosgen, Dialkyl- und Diarylcarbonat. Geeignete aromatische Dicarbonsäuren sind beispielsweise Orthophthaisäure; Terephthalsäure; Isophthalsäure; tert.-Butylisophthalsäure; 3,3'-Diphenyldicarbonsäure; 4,4'-Diphenyldicarbonsäure; 4,4'-Benzophenondicarbonsäure; 3,4'-Benzophenondicarbonsäure; 4,4'-Diphenyletherdicarbonsäure; 4,4'-Diphenylsulfondicarbonsäure; 2,2-Bis(4-carboxyphenyl)propan; Trimethyl-3-phenylindan-4,5-dicarbonsäure.

Ungeachtet des oben angegebenen Zieles, PVC-freie Folien zu schaffen, sei darauf hingewiesen, daß sich mit den speziellen Blockcopolymeren P1 für spezielle Zwecke auch Mischungen mit einer Hartkomponente herstellen lassen, die halogenierte Polymere wie Polyvinylidenchlorid oder Polyvinylchlorid (PVC) enthält. PVC wird bevorzugt modifiziert eingesetzt. Zur Modifizierung werden niedermolekulare Weichmacher (z.B. Dioctylphthalat, Dioctyladipat) und/oder polymere Verbindungen eingesetzt. Mischungen von PVC mit Weichmachern enthalten in der Regel noch Verarbeitungsstabilisatoren (Komponente P3).

Ohne Weichmacher verarbeitbares PVC wird bevorzugt durch (Suspensions-)Pfropfpolymerisation von Vinylchlorid auf einen Elastomer hergestellt. Der Elastomer kann aus Polybutadien-und/oder Polyacrylatkautschuk bestehen.

Weiterhin können eingesetzt werden: Chloriertes Polyethylen; chloriertes Polypropylen; Polyisobutylen; Polyvinylacetat; Polyvinylalkohol; Polyvinylether; Polymethylpenten; Polytetrafluorethylen; Tetrafluorethylen-Perfluorpropylen-Copolymerisate; Copolymerisate von Tetrafluorethylen und Perfluoralkylvinylether ; Ethylen-Tetrafluorethylen-Copolymerisate; Polyvinylidenfluorid; Polyvinylfluorid; Polychlortrifluorethylen; Ethylen-Chlortrifluorethylen-Copolymere.

### Cellulosederivate

Für P2 geeignet sind ferner Cellulose-Abkömmlinge wie Celluloseacetat; Cellulosepropionat; Celluloseacetobutyrat.

### Polyphenylenether

Geeignet als Komponente P2 sind die Polyphenylenether mit dem bekannten Strukturbaustein -[-O-C₆H₄(CH₃)₂-]ₙ-;sie werden bevorzugt durch oxidative Polymerisation unter Kupferkatalyse hergestellt, oder aber auch durch Halogensubstitutionsreaktionen aus den entsprechenden 2,6-Dimethyl-4-halogenphenolen. Sie sind homogen mischbar mit Polystyrol und sehr gut bis homogen mischbar mit den erfindungsgemäßen thermoplastischen Elastomeren. Sie können unmodifiziert oder modifiziert (z.B. mit schlagfestem Polystyrol und/oder Styrol-Butadien-Blockkautschuken) eingesetzt werden. Als Komponente P2 können natürlich auch Polymere Verwendung finden, die der Komponente Pl strukturell ähnlich sind. Beispielsweise können dies lineare und/oder sternförmige Styrol-Butadien-Blockcopolymerisat-Werkstoffe wie Styrolux® (BASF), K-Resin® (Phillips Petroleum) oder Finaclear® (Petrofina) sein.

Als geeignet haben sich auch solche Polymere erwiesen, die einen besonders langen Block A (in der Regel Polystyrolblock A) besitzen und damit als eine Art "Verträglichkeitsvermittler" zwischen Komponente P1 und z.B. den oben genannten linearen und/oder sternförmigen Styrol-Butadien-Blockcopolymerisat-Werkstoffen, Polystyrolen (glasklar oder schlagfest), etc. dienen können.

Als Weichphase können die einschlägig üblichen Kautschuke Verwendung finden. Es können beispielsweise Naturkautschuk, Epichlorhydrin-Kautschuke, Ethylen-Vinylacetat-Kautschuke, Polyethylenchlorsulfonkautschuke, Siliconkautschuke, Polyetherkautschuke, Dienkautschuke, hydrierte Dienkautschuke, Polyalkenamer-Kautschuke, Acrylatkautschuke, Ethylen-Propylen-Kautschuke, Ethylen-Propylen-Dien-Kautschuke, Butylkautschuke und Fluorkautschuke verwendet werden. Bevorzugt wird Acrylatkautschuk, Ethylen-Propylen-(EP-)-Kautschuk, Ethylen-Propylen-Dien (EPDM-)-Kautschuk, insbesondere Butadien-Isopren-Kautschuk, Dienkautschuk oder Siliconkautschuk eingesetzt.

Bei den Acrylatkautschuken handelt es sich i.a. um Alkylacrylat-Kautschuke aus einem oder mehreren C₄-C₈-Alkylacrylaten, wobei bevorzugt mindestens teilweise Butyl-, Hexyl-, Octyl- oder 2-Ethylhexylacrylat verwendet worden sind. Diese Alkylacrylat-Kautschuke können bis zu 30 Gew.-% harte Polymere bildende Monomere wie Vinylacetat, (Meth)acrylnitril, Styrol, substituiertes Styrol, Methylmethacrylat, Vinylether einpolymerisiert enthalten. Die Acrylatkautschuke enthalten weiterhin bis zu 10 Gew.-%, vorzugsweise 1 bis 5 Gew.-% an vernetzend wirkenden, polyfunktionellen Monomeren (Vernetzungsmonomere). Beispiele hierfür sind Monomere, die zwei oder mehr zur Copolymerisation befähigte Doppelbindungen enthalten.

Dien-Kautschuke sind beispielsweise Homopolymerisate von Konjugierten Dienen mit 4 bis 8 Kohlenstoffatomen wie Butadien, Isopren, Piperylen und Chloropren, Copolymerisate solcher Diene untereinander und Copolymerisate solcher Diene mit Styrol-, Acryl- oder Methacrylverbindungen (z.B. Acrylnitril, Methacrylnitril, Acrylsäure, Methacrylsäure, Acrylsäurebutylester, Acrylsäureethylhexylester und Methylmethacrylat). Besonders bevorzugte Dien-Kautschuke sind handelsübliche Butadien-, Butadien-Styrol-, Butadien-Methylmethacrylat-, Butadien-Acrylsäurebutylester- und Butadien-Acrylnitril-Kautschuke (z.B. Cariflex® (Shell); Finaprene® (Fina); Tufprene® (Asahi); Exxellor® (Exxon); Europrene® (Enichem) etc. Die vorstehende Aufzählung und Reihenfolge der genannten Produkte ist willkürlich; die Firmenbezeichnungen sind abgekürzt) .

Geeignete Siliconkautschuke können z.B. vernetzte Siliconkautschuke aus Einheiten der allgemeinen Formeln R₂SiO, RSiO_{3/2}, R₃SiO_{1/2} und SiO_{2/4} sein, wobei R einen einwertigen Rest darstellt. Die Menge der einzelnen Siloxaneinheiten sind dabei so bemessen, daß auf 100 Einheiten der Formel R₂SiO bis 10 Mol-Einheiten der Formel RSiO₃/₂, bis 1,5 Mol-Einheiten R₃SiO_{1/2} und bis 3 Mol-Einheiten SiO_{2/4} vorhanden sind. R kann dabei entweder ein einwertiger gesättigter Kohlenwasserstoffrest mit 1 bis 18 C-Atomen, der Phenylrest oder Alkoxy-Rest oder eine radikalisch leicht angreifbare Gruppe wie der Vinyl- oder der Mercaptopropylrest sein. Bevorzugt ist, daß mindestens 80 % aller Reste R Methyl sind; insbesondere bevorzugt sind Kombinationen aus Methyl und Ethyl oder Phenyl.

Bei der Weichkomponente kann es sich auch um ein mehrstufig aufgebautes Polymerisat handeln (sog. "Kern/Schale-Aufbau", "core/ shell morphology"). Beispielsweise kann ein kautschukelastischer Kern (Glastemperatur T_{G} <0°C von einer "harten" Schale (Polymere mit T_{G} >0°C) oder umgekehrt umhüllt sein.

Die erfindungsgemäßen Komponenten P1 und P2 sind in praktisch jedem beliebigen Mischungsverhältnis herstellbar, z.B. von 0,1 bis 99,9 Gew.-% A, bevorzugt 10 bis 90 Gew.-% P1 und 0,1 bis 99,9 Gew.-% B, bevorzugt 10 bis 90 Gew.- B.

Als Komponente P3) können die erfindungsgemäßen Polymermischungen weitere Zusatzstoffe und Verarbeitungshilfsmittel wie Stabilisatoren, Oxidationsverzögerer, Mittel gegen Wärmezersetzung und Zersetzung durch ultraviolettes Licht, Gleit- und Entformungsmittel, Färbemittel wie Farbstoffe und Pigmente, faser- und pulverförmige Füll- und Verstärkungsmittel, Keimbildungsmittel, Weichmacher usw. enthalten, deren Anteil in der Regel nicht mehr als 70 Gew.-% bevorzugt nicht mehr als 40 Gew.-% beträgt.

Als Beispiele für Oxidationsverzögerer und Wärmestabilisatoren sind Halogenide von Metallen der Gruppe 1 des periodischen Systems, z.B. Natrium-, Kalium- und/oder Lithiumhalogenide, ggf. in Verbindung mit Kupfer-(I)-Halogeniden, z.B. Chloriden, Bromiden, Jodiden, sterisch gehinderte Phenole, Hydrochinone, verschiedene substituierte Vertreter dieser Gruppen und deren Mischungen in Konzentrationen bis zu 1 Gew.-%, bezogen auf das Gewicht der thermoplastischen Formmasse genannt.

Als UV-Stabilisatoren, die im allgemeinen in Mengen bis zu 2 Gew.-%, bezogen auf die Formmasse, verwendet werden, seien verschiedene substituierte Resorcine, Salicylate, Benzotriazole und Benzophenone genannt.

Weiterhin können organische Farbstoffe wie Nigrosin, Pigmente wie Titandioxid, Cadmiumsulfid, Cadmiumselenid, Phthalocyanine, Ultramarinblau und Ruß als Farbstoffe zugesetzt werden, sowie faser- und pulverförmige Füllstoffe und Verstärkungsmittel. Beispiele für letztere sind Kohlenstoffasern , Glasfasern, amorphe Kieselsäure, Asbest, Calciumsilicat (Wollastonit), Aluminiumsilicat, Magnesiumcarbonat, Kaolin, Kreide, gepulverter Quarz, Glimmer und Feldspat. Der Anteil derartiger Füll- und Farbstoffe beträgt im allgemeinen bis zu 50 Gew.-%, bevorzugt bis zu 35 Gew.-%.

Als Keimbildungsmittel können z.B. Talkum, Calciumfluorid, Natriumphenylphosphinat, Aluminiumoxid, Siliciumdioxid und Nylon 22 eingesetzt werden.

Gleit- und Entformungsmittel, welche in der Regel in Mengen bis zu 1 Gew.-% eingesetzt werden können, sind z.B. langkettige Fettsäuren wie Stearinsäure oder Behensäure, deren Salze (z.B. Ca-oder Zn-Stearat) oder Ester (z.B. Stearylstearat oder Pentaerythrittetrastearat) sowie Amidderivate (z.B. Ethylenbisstearylamid). Zur besseren Folienverarbeitung können in Mengen bis zu 0,1 Gew.-% Antiblockmittel auf mineralischer Basis den erfindungsgemäßen Formmassen zugesetzt werden. Als Beispiele seien amorphe oder kristalline Kieselsäure, Calciumcarbonat oder Aluminiumsilikat genannt.

Als Beispiele für Weichmacher seien Phthalsäuredioctylester, Phthalsäuredibenzylester, Phthalsäurebutylbenzylester, Kohlenwasserstofföle, N-(n-Butyl)benzolsulfonamid und o- und p-Tolylethylsulfonamid genannt.

Zur weiteren Verbesserung der Schwerentflammbarkeit können alle für die jeweiligen Thermoplaste bekannten Flammschutzmittel zugegeben werden, insbesondere solche auf Basis von Phosphorverbindungen bzw. roter Phosphor selbst.

Die Herstellung der erfindungsgemäßen Formmassen kann nach an sich bekannten Verfahren erfolgen. Nach einer bevorzugten Ausführungsform erfolgt die Herstellung durch Zugabe der Komponente P₁) sowie gegebenenfalls P3), zur Schmelze der Komponente P₂).

Zweckmäßigerweise verwendet man hierzu Extruder, z.B. Einschnekken- oder Zweischneckenextruder oder andere herkömmliche Plastifizierungsvorrichtungen wie Brabender-Mühlen oder Banbury-Mühlen.

### Beispiele

### Komponente P1

Die Herstellung der Komponente P 1 erfolgte gemäß DE-A 44 20 952:

Ein beheiz- und kühlbarer 50 1-Edelstahlreaktor, der mit einem Kreuzbalkenrührer ausgerüstet war, wurde durch Spülen mit Stickstoff, auskochen mit einer Lösung von sec-Butyllithium und 1,1-Diphenylethylen in Cyclohexan (Molverhältnis 1:1) und trocknen vorbereitet.

Anschließend wurden 22,8 1 Cyclohexan eingefüllt und 42 ml s-Butyllithium als Initiator, 65,8 ml Tetrahydrofuran und Styrol (S) und Butadien (B) in den in der nachstehenden Tabelle 1 angegebenen Mengen (g) und nach dem angegebenen Zeittaktprogramm zugesetzt. Angegeben ist auch die Polymerisationsdauer t in Minuten sowie Anfangs- und Endtemperatur T_{A} bzw. T_{E} (in OC), wobei darauf hinzuweisen ist, daß die Polymerisationsdauer stets groß gegen die Dauer des Monomerzulaufs war.

Die Temperatur des Reaktionsgemisches wurde durch Heizung oder Kühlung des Reaktormantels gesteuert. Nach Umsetzungsende (Verbrauch der Monomeren) wurde mit Ethanol, bis zur Farblosigkeit titriert und die Mischung mit einem 1,5-fachen Überschuß an Ameisensäure sauer gestellt. Zuletzt wurden 0,5 Gew.-% eines handelsüblichen Stabilisators 2-t-butyl-6-(3-t-butyl-2-hydroxy-5-methylbenzyl)-4-methylphenylacrylat (Irganox^{®} 3052; Ciba-Geigy, Basel) und 0,85 Gew.-% Trisnonylphenylphosphit zugesetzt.

Die erhaltene Polymerlösung wurde vom Lösungsmittel befreit, mit 0,5 Gew.-% eines Gleitmittels (Polyethylen-Abbauwachs mit einer Erweichungstemperatur von 95°C; BeSquare® 195; Petrolite) versetzt, in einer Knetmaschine (Werner u. Pfleiderer; ZSK 30) entgast und granuliert.

**Tabelle 1**

| | Komponente P1 |
|---|---|
| THF (ml) | 65,8 |
| s-BuLi (ml) | 42 |
| Styrol 1 (g) | 1008 |
| T(A)/T(E) (°C) | 30/70 |
| Zeit (min) | 30 |
| Butadien 1 (g) | 1120 |
| Styrol 2 (g) | 1412 |
| T(A)/T(E) (°C) | 68/96 |
| Zeit (min) | 17 |
| Butadien 2 (g) | 1120 |
| Styrol 3 (g) | 1412 |
| T(A)/T(E) (°C) | 60/84 |
| Zeit (min) | 12 |
| Butadien 3 (g) | 1120 |
| Styrol 4 (g) | 1412 |
| T (A)/T(E) (°C) | 64/83 |
| Zeit (min) | 6 |
| Styrol 5 (g) | 1008 |
| T(A)/T(E) (°C) | 70/76 |
| Zeit (min) | 14 |
| Mn (g/mol) | 107 000 |
| Mp (g/mol) | 141 000 |
| Mw (g/mol) | 159 000 |

Zum Vergleich wurden folgende Komponenten eingesetzt:
- P/1V: Weich-PVC bestehend aus
63 Gew.-% PVC (Vinoflex® 57114, BASF AG),
35 Gew.-% Diethylhexylphthalat
2 Gew.-% Mischung 1:1 Calciumstearat mit Zinkstearat
- P/2V: Styrol-Butadien-Styrol-Dreiblockcoplymer mit einem Styrolgehalt von 30 Gew.-%; MVR (200°C/5 kg):1 ml/10' (Cariflex® TR 1101 der Firma Shell)
- P/3V: Styrol-Butadien-Styrol-Dreiblockcopolymer mit einem Styrolgehalt von 30 Gew.-%; MVR (200°C/5 kg): 6 ml/10' (Cariflex® TR 1102 der Firma Shell)
- P/4V: Radiales Styrol-Butadien-Blockcopolymer, erhältlich gemäß EP-A2 390 019 (Komponente A1); (Styrolux^{®} 684 D der BASF AG)

### Herstellung der Formteile

Die erfindungsgemäßen Formteile sowie die zu Vergleichszwecken hergestellten Formteile wurden mittels Schlauchprofilextrusion auf einem Barmag-Einschneckenextruder (Schneckendurchmesser 45 mm) bei einer Massetemperatur von 220°C und einer Düsentemperatur von 230°C hergestellt. Als Extrusionshilfsmittel (Gleitmittel) wurde bei den Vergleichskomponenten (P/2V bis P/4V und der erfindungsgemäßen Mischung jeweils 0,1 Gew.-% Calciumstearat eingesetzt. Es wurde mit einem Durchsatz von 3 bis 4 kg/h extrudiert. Das Schlauchprofil wurde mit einem Düsendurchmesser von 5,5 mm und einem Dorndurchmesser von 3,7 mm derart eingestellt, daß der extrudierte Schlauch durch eine nachfolgende Verstreckeinrichtung auf einen Außendurchmesser von 4 mm und einen Innendurchmesser von 2,7 mm "gestreckt" wurde. Gekühlt wurde mit einem wasserdurchflossenen 6,7 mm starken Kalibrierring.

### Folgende Prüfungen wurden an den Schläuchen durchgeführt:

a) Memory-Effekt:
Ein Schlauchstück von 50 cm Länge wurde um 400 % verstreckt und sofort wieder entspannt. Anschließend wurde gemessen, welche Restverstreckung nach einer Minute verbleibt. Die angegebenen Werte sind Mittelwerte aus in Längs- und Querrichtung gemessenen Werten. Folgende Bewertungsskala wurde eingeführt:

| Restverstreckung | Note |
|---|---|
| 0-10 % | 1 |
| 11-30 % | 2 |
| 31-60 % | 3 |
| 61-90 % | 4 |
| über 90 % | 5 |

b) Transparenz:
Die Transparenz der Schläuche wurde visuell bewertet (1 = sehr gut, 5 = sehr schlecht).
c) Druckfestigkeit:
Ein verschlossenes Schlauchstück von 50 cm Länge wurde mit einer Pumpe mit einem Wasserdruck von 3 bar beaufschlagt und die Zeit bestimmt, nach der der Druck auf die Hälfte abgefallen war.
d) Knickfestigkeit:
Schlauchstücke geeigneter Länge wurden auf einen Biegeradius von 2 cm gebogen und beobachtet, ob die Biegestelle abknickt (1 = kein Knick; 3 = leichter Knick, aber noch Restschlauchvolumen offen; 5 = Knick hat Schlauch komplett abgeschlossen oder Schlauch ist beim Abknicken gebrochen).
Die Ergebnisse der Messungen sind der Tabelle 2 zu entnehmen.

**Tabelle 2**

| | Beispiel | Vergl.1 | Vergl.2 | Vergl.3 | Vergl.4 |
|---|---|---|---|---|---|
| Schlauchmaterial | P1 | P/1V | P/2V | P/3V | P/4V |
| Memory-Eff. | 1 | 1-2 | -*) | 1**) | 5 (Bruch) |
| Transparenz | 1 | 1 | - | 5 | 2 |
| Druckf. | >48h | >48h | - | 1 h | >48h |
| Knickf. | 1 | 1 | - | 1 | 5 (Bruch) |

| | | | | | |
|---|---|---|---|---|---|
| *) Material verbrannt - nicht extrudierbar | | | | | |
| **) Material braun verfärbt; Gelpartikel (vernetzt) | | | | | |

## Patentansprüche

1. Medizintechnisches Formteil, insbesondere Bauteil für ein Übertragungssystem für Infusions- oder Transfusionszwecke, aus einem Polymeren P1 oder einer Mischung aus, bezogen auf die Gewichtssumme aus P1 und P2,
P1: 0.1-100 Gew. % eines durch anionische Polymerisation erhaltenen kautschukelastischen Blockcopolymerisats Pl aus mindestens einem einpolymerisierte Einheiten eines vinylaromatischen Monomeren aufweisenden, eine Hartphase bildenden Block S und mindestens einem sowohl einpolymerisierte Einheiten eines vinylaromatischen Monomeren (S) wie eines Diens (B) aufweisenden elastomeren, eine Weichphase bildenden Block B/S, wobei die Polymerisation der Weichphase in Gegenwart eines polaren Cosolvens vorgenommen worden ist, derart, sie ein statistisches Copolymerisat des vinylaromatischen Monomeren mit dem Dien bildet und wobei die Glastemperatur T_{g} des Blocks S über 25°C und die des Blocks B/S unter 25°C liegt und das Phasenvolumen-Verhältnis von Block S zu Block B/S so gewählt ist, daß der Anteil der Hartphase am gesamten Blockcopolymerisat 1 bis 40 Volumen-% und der Gewichtsanteil des Diens weniger als 50 Gew.% beträgt
P2: gegebenenfalls bis zu 99.9 % eines thermoplastisch verarbeitbaren und/oder duroplastischen Polymeren P2.

2. Formteil nach Anspruch 1, enthaltend
P1: 1 bis 99 Gew.-% des kautschukelastischen Blockcopolymerisats P1,
P2 1 bis 99 Gew.-% des thermoplastisch verarbeitbaren Polymeren P2.

3. Formteil nach Anspruch 1, enthaltend
P1: 50 bis 99 Gew.-% des kautschukelastischen Blockcopolymerisats P1,
P2 1 bis 50 Gew.-% des thermoplastisch verarbeitbaren polymeren P2.

4. Formteil nach Anspruch 1, dadurch gekennzeichnet, daß Tg der Hartphase S des Blockcopolymerisats P1 über 50°C und Tg der Weichphase B/A unter 5°C liegt.

5. Formteil nach Anspruch 1, dadurch gekennzeichnet, daß das vinylaromatische Monomere des Blockcopolymerisats P1 ausgewählt ist aus Styrol, Å-Methylstyrol, Vinyltoluol und Diphenylethylen und das Dien aus Butadien und Isopren.

6. Formteil nach Anspruch 1, dadurch gekennzeichnet, daß das Blockcopolymerisat P1 mehrere Blöcke B/S mit unterschiedlicher Molmasse je Molekül aufweist.

7. Formteil nach Anspruch 1, dadurch gekennzeichnet, daß das Blockcopolymerisat P1 mehrere Blöcke S mit unterschiedlicher Molmasse je Molekül aufweist.

8. Formteil nach Anspruch 1, enthaltend ein Blockcopolymerisat P1, dargestellt durch eine oder mehrere der allgemeinen Formeln (1) bis (11)
(1) (S-B/S)ₙ;
(2) (S-B/S)ₙ-S;
(3) B/S-(S-B/S)ₙ;
(4) X-[(S-B/S)ₙ]ₘ₊₁;
(5) X-[(B/S-S)ₙ]ₘ₊₁;
(6) X-[(S-B/S)ₙ-S]ₘ₊₁;
(7) X-[(B/S-S)ₙ-B/S]ₘ₊₁;
(8) Y-[(S-B/S)ₙ]ₘ₊₁;
(9) Y-[(B/S-S)ₙ]ₘ₊₁;
(10) Y-[(S-B/S)ₙ-S]ₘ₊₁;
(11) Y-[(B/S-S)ₙ-B/S]ₘ₊₁;
wobei S für den vinylaromatischen Block und B/S für den statistisch aus Dien- und vinylaromatischen Einheiten steht, X den Rest eines n-funktionellen Initiators, Y den Rest eines m-funktionellen Kopplungsmittels und m und n natürliche Zahlen von 1 bis 10 bedeuten.

9. Formteil nach Anspruch 8, enthaltend ein Blockcopolymerisat mit einer der allgemeinen Formeln S-B/S-S, X-[B/S-S]₂ und Y-[B/S-S]₂.

10. Formteil nach Anspruch 1, enthaltend ein Blockcopolymerisat, dessen Weichphase unterteilt ist in Blöcke
(12) (B/S)₁-(B/S)₂ ,
(13) (B/S)₁-(B/S)₂-(B/S)₁
oder
(14) (B/S)₁-(B/S)₂-(B/S)₃,
wobei die Indices 1, 2, 3... unterschiedliche Strukturen in dem Sinne bedeuten, daß das Vinylaromat/Dien-(S/B)-Verhältnis in den einzelnen Blöcken B/S unterschiedlich ist oder sich innerhalb eines Blocks In den Grenzen (B/S)₁≤(B/S)₂ kontinuierlich ändert, wobei die Glasübergangstemperatur T_{g} jedes Teilblocks unter 25°C liegt.

11. Verwendung eines Formteils nach einem der Ansprüche 1 bis 10 in einem Übertragungssystem für Infusions- oder Transfusionszwecke.

## Claims

1. A medical molding, in particular a component for a transfer system for infusion or transfusion purposes, comprising a polymer P1 or a mixture of, based on the total weight of P1 and P2,
P1: from 0.1-100% by weight of an elastomeric block copolymer P1 obtained by anionic polymerization comprising at least one block S which contains polymerized units of a vinylaromatic monomer and forms the hard phase and at least one elastomeric block B/S which contains polymerized units of both a vinylaromatic monomer (S) and a diene (B) and forms the soft phase, the polymerization of the soft phase having been carried out in the presence of a polar cosolvent in the manner such that it forms a random copolymer of the vinylaromatic monomer with the diene, and the glass transition temperature T_{g} of the block S being above 25°C and that of the block B/S below 25°C and the phase volume ratio of block S to block B/S being chosen so that the amount of the hard phase in the total block copolymer is from 1 to 40% by volume and the amount by weight of the diene is less than 50% by weight, and
P2: if required, up to 99.9% by weight of a polymer P2 which can be processed by a thermoplastic method and/or is thermosetting.

2. A molding as claimed in claim 1, containing
P1: from 1 to 99.9% by weight of the elastomeric block copolymer P1 and
P2: from 1 to 99% by weight of the polymer P2 processible by a thermoplastic method.

3. A molding as claimed in claim 1, containing
P1: from 50 to 99% by weight of the elastomeric block copolymer P1 and
P2: from 1 to 50% by weight of the polymer P2 processible by a thermoplastic method.

4. A molding as claimed in claim 1, wherein the T_{g} of the hard phase S of the block copolymer P1 is over 50°C and the T_{g} of the soft phase B/S is less than 5°C.

5. A molding as claimed in claim 1, wherein the vinylaromatic monomer of the block copolymer P1 is selected from styrene, α-methylstyrene, vinyltoluene and diphenylethylene, and the diene from butadiene and isoprene.

6. A molding as claimed in claim 1, wherein the block copolymer P1 has a plurality of blocks B/S having different molar masses per molecule.

7. A molding as claimed in claim 1, wherein the block copolymer P1 has a plurality of blocks S having different molar masses per molecule.

8. A molding as claimed in claim 1, containing a block copolymer P1 of one or more of the formulae (1) to (11)
(1) (S-B/S)ₙ;
(2) (S-B/S)ₙ-S;
(3) B/S-(S-B/S)ₙ;
(4) X-[(S-B/S)ₙ]ₘ₊₁;
(5) X-[(B/S-S)ₙ]ₘ₊₁;
(6) X-[(S-B/S)ₙ-S]ₘ₊₁;
(7) X-[(B/S-S)ₙ-B/S]ₘ₊₁;
(8) Y-[(S-B/S)ₙ]ₘ₊₁;
(9) Y-[(B/S-S)ₙ]ₘ₊₁;
(10) Y-[(S-B/S)ₙ-S]ₘ₊₁;
(11) Y-[(B/S-S)ₙ-B/S]ₘ₊₁;
where S is the vinylaromatic block and B/S is the block composed of random diene and vinylaromatic units, X is the radical of an n-functional initiator, Y is the radical of an m-functional coupling agent and m and n are natural numbers from 1 to 10.

9. A molding as claimed in claim 8, containing a block copolymer of one of the formulae S-B/S-S, X-[-B/S-S]₂ and Y-[-B/S-S]₂.

10. A molding as claimed in claim 1, containing a block copolymer whose soft phase is divided into blocks
(12) (B/S)₁-(B/S)₂,
(13) (B/S)₁-(B/S)₂-(B/S)₁
or
(14) (B/S)₁-(B/S)₂-(B/S)₃,
where the indices 1, 2 and 3 denote different structures in the sense that the vinylaromatic/diene (S/B) ratio in the individual blocks B/S is different or changes continuously within a block within the limits (B/S)₁≤(B/S)₂, wherein the glass transition temperature T_{g} of each part-block is less than 25°C.

11. The use of a molding as claimed in any of claims 1 to 10 in a transfer system for infusion or transfusion purposes.

## Revendications

1. Elément moulé à usage médical, en particulier élément de structure pour un système de transfert à des fins de perfusion ou de transfusion, constitué par un polymère P1 ou par un mélange, rapporté à la somme pondérale de P1 et de P2, de
P1: à concurrence de 0,1 à 100% en poids, un copolymère séquence P1 manifestant une élasticité analogue à celle du caoutchouc, obtenu par polymérisation anionique, constitué par au moins une séquence S formant une phase dure, présentant des motifs d'un monomère vinylaromatique introduits par polymérisation et par au moins une séquence élastomère BIS formant une phase tendre, présentant des motifs aussi bien d'un monomère vinylaromatique S que d'un diène B, introduits par polymérisation, dans lequel la polymérisation de la phase tendre a été réalisée en présence d'un cosolvant polaire de telle sorte qu'elle forme un copolymère statistique du monomère vinylaromatique avec le diène et dans lequel la température de transition vitreuse T_{g} de la séquence S est supérieure à 25°C, celle de la séquence B/S étant inférieure à 25°C, et le rapport volumique des phases de la séquence S à la séquence B/S est sélectionné de telle sorte que la fraction de la phase dure du copolymère séquence total représente de 1 à 40% en volume et la fraction pondérale du diène est inférieure à 50% en poids,
P2: le cas échéant, jusqu'à concurrence de 99,9%, un polymère P2 apte à un traitement thermoplastique et/ou thermodurcissable.

2. Elément moulé selon la revendication 1, contenant
P1: à concurrence de 1 à 99% en poids, le copolymére séquencé P1 manifestant une élasticité analogue à celle du caoutchouc,
P2: à concurrence de 1 à 99% en poids, le polymère P2 apte à un traitement thermoplastique.

3. Elément moulé selon la revendication 1, contenant
P1: à concurrence de 50 à 99% en poids, le copolymère séquence P1 manifestant une élasticité analogue à celle du caoutchouc,
P2: à concurrence de 1 à 50% en poids, le polymère P2 apte à un traitement thermoplastique.

4. Elément moulé selon la revendication 1, caractérisé en ce que la T_{g} de la phase dure S du copolymère séquence P1 est supérieure à 50°C et la T_{g} de la phase tendre BIS est inférieure à 5°C.

5. Elément moulé selon la revendication 1, caractérisé en ce que le monomère vinylaromatique du copolymère séquence P1 est choisi parmi le groupe constitué par le styrène, l'alpha-méthylstyrène, le vinyltoluène et le diphényléthylène, le diène étant choisi parmi le groupe constitué par le butadiène et l'isoprène.

6. Elément moulé selon la revendication 1, caractérisé en ce que le copolymère séquencé P1 présente, par molécule, plusieurs séquences B/S possédant des masses molaires différentes.

7. Elément moulé selon la revendication 1, caractérisé en ce que le copolymère séquence P1 présente, par molécule, plusieurs séquences S possédant des masses molaires différentes.

8. Elément moulé selon la revendication 1, contenant un copolymère séquence P1 représenté par une ou plusieurs des formules générales (1) à (11)
(1) (S-B/S)ₙ;
(2) (S-B/S)ₙ-S;
(3) B/S-(S-B/S)ₙ;
(4) X-[(S-B/S)ₙ]ₘ₊₁;
(5) X-[(B/S-S)ₙ]ₘ₊₁;
(6) X-[(S-B/S)ₙ-S]ₘ₊₁;
(7) X-[(B/S-S)ₙ-B/S]ₘ₊₁;
(8) Y-[(S-B/S)ₙ)ₘ₊₁;
(9) Y-[(B/S-S)ₙ]ₘ₊₁;
(10) Y-[(S-B/S)ₙ-S]ₘ₊₁;
(11) Y-[(B/S-S)ₙ-B/S]ₘ₊₁
dans lesquelles S représente la séquence vinylaromatique et B/S représente la séquence statistique constituée par des motifs diéniques et vinylaromatiques, X représente le radical d'un initiateur n-fonctionnel, Y représente le radical d'un agent de couplage m-fonctionnel et m et n représentent des nombres naturels de 1 à 10.

9. Elément moulé selon la revendication 8, contenant un copolymère séquencé répondant à une des formules générales S-B/S-S, X-[B/S-S]₂ et Y-[B/S-S]₂.

10. Elément moulé selon la revendication 1, contenant un copolymère séquencé dont la phase tendre est subdivisée en séquences
(12) (B/S)₁-(B/S)₂;
(13) (B/S)₁-(B/S)₂-(B/S)₁
ou
(14) (B/S)₁-(B/S)₂-(B/S)₃
dans lesquelles les indices 1, 2, 3 ... représentent différentes structures dans le sens où le rapport composé vinylaromatique/diène (S/B) dans les séquences individuelles B/S est différent ou se modifie en continu à l'intérieur d'une séquence dans les limites (B/S)₁≤(B/S)₂, la température de transition vitreuse T_{g} de chaque séquence partielle étant inférieure à 25°C.

11. Utilisation d'un élément moulé selon l'une quelconque des revendications 1 à 10 dans un système de transfert à des fins de perfusion ou de transfusion.
